Europäisches Patentamt

European Patent Office

Office européen des brevets

(11) Publication number: **0 264 185**
**A2**

# EUROPEAN PATENT APPLICATION

(21) Application number: 87307823.2

(22) Date of filing: 04.09.87

(51) Int. Cl.⁴: **G01N 33/74** , G01N 33/566 , G01N 33/574 , C12Q 1/68

(30) Priority: 29.09.86 US 913155

(43) Date of publication of application:
20.04.88 Bulletin 88/16

(84) Designated Contracting States:
CH DE FR GB LI SE

(71) Applicant: **BAYLOR COLLEGE OF MEDICINE**
**One Baylor Plaza**
**Houston, TX 77030(US)**

(72) Inventor: **Hutchens, Thomas William**
**5502 Cheltenham**
**Houston Texas 77096(US)**

(74) Representative: **Lucas, Brian Ronald et al**
**Lucas, George & Co. 135 Westhall Road**
**Warlingham Surrey CR3 9HJ(GB)**

(54) Urea-induced DNA-binding assay for quantitative determination of estrogen receptor proteins.

(57) A quantitative determination of estrogen receptor protein concentrations in normal and cancerous tissues is described using urea to specifically promote adsorption of estrogen-receptor complexes to DNA. There is little or no interference from non-receptor steroid-binding proteins or free estrogen or ligand.

## Urea-Induced DNA-Binding Assay for Quantitative Determination of Estrogen Receptor Proteins.

The present invention relates generally to a method for determining estrogen receptor protein concentrations in normal and cancerous tissues. More specifically, the invention relates to the detection of the estrogen receptors by a direct assay using DNA to absorb estrogen-receptor complexes in the presence of urea.

It has been recommended that estrogen receptor proteins be quantified in all tumours from patients with breast cancer (1-3) or endometrial adenocarcinoma (4,5). Thus, both primary cancers and metastatic lesions are routinely assayed for these receptor proteins. This type of information provides a guide for predicting patient response to hormonal manipulation and the probability of a disease-free interval (1-7).

Currently the accepted practice for determining the concentration of functional estrogen-receptor concentrations in normal and tumourous tissue extracts is dependent upon the use of charcoal solutions or hydroxylapatite solutions to separate steroid-receptor complexes from free steroid (8,9) or alternatively both charcoal and sucrose density gradient centrifugation (10). The assay techniques for the estrogen receptor include the dextran-coated charcoal (DCC) steroid adsorption procedure and sucrose density gradient centrifugation (SDG). These procedures are based. upon the steroid-specific, high-affinity interaction of radiolabelled estrogen (i.e. $^3$H-estradion-17$\beta$ or $^{125}$I-16$\alpha$iodestradiol-17$\beta$) with the low quantity of receptor present (femtomoles of estrogen receptor/mg cytosol protein) in the solublized portion of the normal or tumor tissue extracts (cytosol) (10). These assays provide an indirect measurement of steroid receptors. The amount of receptor proteins is determined by calculating the difference between the total bound estrogen and the non-specific protein bound estrogen. Both the SDG technique and the DCC procedure are cumbersome, labour-intensive, difficult to control procedures that lack specificity- and require considerable equipment and expertise.

There have been many scientific investigations and publications devoted to simplifying these procedures and ensuring their quality control (11-13). These efforts have met with little or no success. A major stumbling block has been the lack of significant advances in the separation of the estrogen-receptor complex from free estrogens and non-specific protein (non-receptor) estrogen binding. The non-specific binding can comprise as much as 95% of the total binding and can result in a high estimate of estrogen receptor concentration.

Another severe limitation of the DDC and SDG assay procedures is the requirement for a minimum overall cytosol protein concentration of at least 1-2 mg protein per ml of cytosol. The estrogen receptors cannot be accurately quantified at lower protein concentrations because of non-specific DCC protein-adsorption artifacts. This is further complicated by the small volumes (i.e. 0.05-0.20 ml) which must be used in current SDG procedures. When the cytosol protein concentrations are too low, for example, a small tumor or small sample biopsy, the assay either cannot be performed or a more sophisticated indirect procedure, the hydroxylapatite adsorption assay (HAP), must be employed (14,15). A serious limitation of the HAP assay is the background "noise" created by non-specific adsorption of free steroid and/or non-specific estrogen binding proteins. During HAP washing periods to reduce background noise, some estrogen-receptor complexes may be lost resulting in a low. estimate of estrogen receptor concentration. Thus, this assay is not recognized for routine analysis and is only used by a few testing centers when the tissue sample is too small for the other procedures.

The steroid-induced DNA binding properties of the estrogen receptor have been known for many years (16). Previous investigators have found that the cytosolic estrogen-receptor complex binds to DNA after transient exposure to elevated temperature (i.e. 30 °C for 30 min) or elevated ionic strengths (i.e. 0.4-0.6 M KCl for 30 min).

TABLE A - ESTROGEN RECEPTOR INTERACTION WITH DNA
COMPARISON OF CONDITIONS UTILIZED TO PROMOTE·INTERACTION
BETWEEN ESTROGEN RECEPTOR (ER) AND DNA

| CONDITIONS OF EXPOSURE OF ER | | % ER BOUND TO DNA |
|---|---|---|
| 1. | KCL | 20% |
| 2. | ELEVATED TEMP. | 12-40% |
| 3. | UREA | 95% |

From Table A it is clear that urea induced binding is far superior.

Other traditional methods of transforming or activating the estrogen-receptor complex included extensive dilution or purification. These procedures yield only partial receptor-DNA interaction. Furthermore, all of the previous characterizations of estrogen receptor-DNA interaction have been ascribed to weak, electrostatic-type interactions. Although it has been speculated that the determination of a functional DNA binding site on the receptor may be helpful in predicting the response of an endocrine tumour to hormonal therapy, the existing procedures for measuring receptor-DNA interactions are both not adequate and not reproducible.

Another assay under development is the monoclonal antibody (MAB) assay. Thus far it has not sufficiently correlated with the existing biochemical assays (i.e. DCC, HAP, SDG) for the accurate quantification of estrogen receptor. Furthermore, since hormonal therapy is correlated with functional receptors, there is considerable doubt about the acceptability of any receptor assay, particularly MAB, which does not measure or reflect receptor function (i.e. steroid binding or DNA binding properties). Non-specific, false-positive values are consistently detected using the MAB assay.

From the above discussion it should be apparent that the DCC, SDG, HAP and MAB assays for estrogen receptors have many limitations. These include:

(1) All are indirect assays for the estrogen·receptor since the components not only bind the receptor but also bind non-specifically to other proteins and unbound ligand. Thus to measure the amount of receptors requires parallel procedures to quantify the total binding and the non-specific binding. The calculation:

Receptor binding = total binding - non-specific binding .

must be performed to interpret estrogen receptor concentrations. The indirect nature of the assay frequently represents a source of error in the clinical laboratory;

(2) The DCC and HAP assays offer extremely variable performance due to irreproducible supplies of charcoal or hydroxylapatite respectively. This irreproducibility leads to variable efficiencies in steroid adsorption, requiring careful and routine monitoring;

(3) The DCC assay is limited to cytosol protein concentrations above 1-2 mg/ml to prevent non-specific adsorption or denaturation of receptor proteins;

(4) The HAP assay requires extensive experience and/or skill to use routinely; and finally,

(5) The SDG procedure is long (normally requiring 2 days), labour intensive, requires highly skilled personnel and expensive equipment and cannot be used with larger volumes (>0.2 ml) of diluted cytosol unless the receptor concentrations (usually unknown before the assay) are very high.

Attempts to simplify or improve the DCC, HAP and SDG assays have not been successful. Thus, there exists a need for an improved means for determining estrogen-receptor concentrations in normal and cancerous tissues.

REFERENCES

1. Fisher B, Redmond C, Brown A, et al. (1981) Treatment of primary breast cancer with chemotherapy and tamoxifen. N Engl J Med 305, 1-6.

2. Fisher B, Redmond C, Brown A, et al. (1983) Influence of tumour estrogen and progesterone receptor levels on the response to tamoxifen and chemotherapy in primary breast cancer, J Clin Oncol 1,227-241.

3. Proceedings of the NIH Concensus Development Conference on Steroid Receptors in Breast Cancer. (1980) Cancer 46(12), 2759-2963.

4. Ehrlich CE, Young PCM, Cleary RE. (1981) Cytoplasmic progesterone and estradiol receptors in normal, hyperplastic, and carcinomatous endometria: therapeutic implications. Am J Obstet Gynecol 141, 539-546.

5. Martin JD, Hahnel R, McCartney AJ, Woodings TL. (1983) The effect of estrogen receptor status on survival in patients with endometrial cancer. Am J Obstet Gynecol 147, 322-324.

6. Rose C, Mouridsen HT. Combined cytotoxic and endocrine therapy in breast cancer. Progress in Cancer Research and Therapy, Vol. 31, F. Bresciani, RJB King, ME Lippman, N Namer and J-P Raynaud, eds., Raven Press, New York, NY, 1984, pp 269-286.

7. Allegra JC, Kubota TT. Combination chemo-hormonal therapy in patients with stage IV breast cancer. Progress in Cancer Research and Therapy, Vol. 31, F. Bresciani, RJB King, ME Lippman, N Namer and J-P Raynaud, eds., Raven Press, New York, NY, 1984, pp 287-291.

8. Korenman SG, Perrin LE, McCallum TP. (1969) A radio-ligand binding assay system for estradiol measurement in human plasma. J. Clin. Endocrinol. 29:879-883.

9. Mester J, Robertson DM, Feherty P, Kellie AE. (1970) Determination of high-affinity oestrogen receptor sites in uterine supernatant preparations. Biochem. J. 120:831-836.

10. Toft D, Gorski J. (1966) A receptor molecule for estrogens: Isolation from the rat uterus and preliminary characterization. Proc. Natl. Acad. Sci. USA 55:1574-1581.

11. Chamness GC, K, McGuire WL, (1975) Scatchard plots: Common errors in correction and interpretation. Steroids 25:538-542.

12. Peck EJ Jr, Clark JH. (1977) Effect of ionic strength on charcoal absorption assays of receptor estradiol complexes. Endocrinology 101:1034-1043.

13. Wittliff JL, Durant JR, Fisher B. (1981) Methods of Steroid Receptor Analyses and Their Quality Control in the Clinical Laboratory in Progress in Clinical and Biological Research 74:397-411.

14. Erdos T, Best-Belpomme M, Bassada R. (1970) A rapid assay for binding estradiol to uterine receptor(s). Anal. Biochem. 37:244-252.

15. Pavlik EJ, Coulson PB. (1976) Hydroxylapatite "batch" assay for estrogen receptors: increased sensitivity over present receptor assays. J. Steroid Biochem. 7(5):357-68.

16. Milgrom, E. (1981) Activation of Steroid Receptor Complexes. Biochemical Actions of Hormones, 8:465-492.

SUMMARY OF THE INVENTION

It is, therefore, an object of at least preferred embodiments of the present invention to produce a method which directly measures the functional estrogen-receptor. Another object of at least preferred embodiments of this invention is to develop· an assay, the UDB assay, which is rapid, simpler and more accurate than current methods.

Still another object of at least preferred embodiments of the present invention is to provide type assay which is one of the simplest ligand binding laboratory procedures available.

It is a further object of at least preferred embodiments of the present invention to provide an assay for very dilute cytosol protein concentrations, i.e., <1 mg/ml, thus allowing the assay to be used with small tumour biopsy specimens.

Furthermore, it is an object of at least preferred embodiments of the present invention to provide an assay which is less prone to error than currently utilized assays.

Thus, in attempting to accomplish these objectives, there is provided in accordance with one aspect of the present invention, a direct assay for the detection of estrogen receptors, comprising of the steps of adding estrogen receptor ligand-estrogen receptor complex, DNA and urea in amounts sufficient to form a mixture comprising a ligand-receptor-DNA complex, and determining the amount of the ligand-receptor-DNA complex as an indication of the amount of estrogen receptors. Preferably, cytosol from endocrine target tissue is combined with an estrogen receptor ligand in amounts sufficient to form the ligand-receptor complex. Advantageously, the DNA is selected from the group consisting of free single stranded DNA, immobilized single stranded DNA, free double stranded DNA and immobilized double stranded DNA. When the DNA is one of the free DNAs, the assay comprises the further step of passing the mixture through a DNA binding filter to separate the ligand-receptor-free DNA complex.

The estrogen receptor ligand is, preferably, estrogens, estradiol, iodoestradiol, anti-estrogens, diethylstilbestrol, tamoxifen, fluorescein-linked estradiol, and most preferably, radiolabelled estradiol.

In a particularly desirable embodiment, the direct assay comprises the steps of incubating cytosol from endocrine target tissue with radiolabelled-estradiol in amounts sufficient to form an estradiol-estrogen receptor complex adding urea and DNA in amounts sufficient to form an estradiol-estrogen receptor-DNA complex and determining the amount of the estradiol-estrogen receptor-DNA complex as an indication of the amount of estrogen receptors. Preferably, according to this assay, the cytosol comprises about 20 mg of protein/ml the amount of radiolabelled estradiol is at least about 10 nM the incubation is at about 0°C for at least about 90 minutes and the urea is in a concentration of at least about 2 M.

The urea-promoted DNA-binding assay (UDB) for estrogen-receptor complexes according to at least preferred embodiments of the present invention is a direct assay for the estrogen receptor. Furthermore, the UDB measures both the steroid binding and DNA binding properties of the receptors. Additionally, since the ligand-receptor-DNA interaction is specific, there is no detectable interference from free radiolabelled steroid or non-specific estrogen binding proteins. This low background noise results in increased sensitivity even with very low protein concentration. By eliminating the parallel assay to compensate for interference there is a significant decrease in time, expense, calculation and interpretation. Furthermore, these improvements greatly reduce the potential for error in measurement.

The assay of at least preferred embodiments of the present invention results in a very high and specific affinity of estrogen-receptor complexes for DNA in the presence of urea. Accordingly, the assay is less prone to error than currently utilized assays.

It is a further advantage of at least preferred embodiments of the present invention that the mode of DNA immobilization and sequence of procedures allows several options for the accommodation of this assay to individual laboratories and for the automation of this assay.

A further advantage of this invention is that UDB measures both the steroid binding and DNA-binding function in a single procedure. This advantage is not possible with existing alternative assays.

Other further objects, features, and advantages will be apparent from the following description of preferred embodiments of the invention.


## DESCRIPTION OF THE PREFERRED EMBODIMENTS

### Experimental Procedure:

Materials: Endocrine target tissue is obtained from breast and uterine tissue and breast and uterine tumours. The sample can be used fresh or can be frozen in liquid nitrogen and stored at -85°C. $^3$H-estradiol-17$\beta$, specific activity 90-101 Ci/m moles, was purchased from New England Nuclear. $^{125}$I-iodestradiol-17$\beta$, specific activity 1600-2000 Ci/m mole, was obtained from New England Nuclear. Ultra-pure urea, enzyme grade, was obtained from Bethesda Research Labs. DNA was obtained from Sigma Chemical Co. and Bethesda Research Labs. Prepared Sephadex G-25 (PD-10) columns 6 cm $^\times$ 1.5 cm were obtained from Pharmacia. All other materials are commercially available from chemical and scientific suppliers.


### Preparation of Cytosol and Labelling of Estrogen Receptors

Functional estrogen receptor proteins are quantified directly according to the following procedures. All procedures were performed at 0.6 °C or in ice. The procedures can easily be performed in a cold room.

Fresh or frozen endocrine target tissue is homogenized (blended) with homogenizing buffer (10-25 mM Tris-HCl containing 1.5 mM disodium ethylenediaminetetraacetic acid (EDTA), nM dithiothreitol (DTT) and 10-20% glycerol, pH 7.4 at 0°C). The endocrine target tissue is usually biopsy material from breast and/or uterine tissues however, metasticies in other organs have been used to measure the estrogen receptors to direct therapy. The tissue can be normal or tumourous. The homogenization can be performed with any homogenizing, pulverizing, blending or cellular disrupting methods. The ratio of buffer to tissue is not critical, however, the preferred method is to combine 2-8 volumes of buffer. This is about 4 ml buffer/gm tissue.

The endocrine target tissue homogenate is centrifuged at about 100,000 × gravity for about 60 minutes to sediment or pellet all particulate cell matter. The supernatant fluid at this point is essentially particle free and is termed cytosol. The estrogen receptors, if present, are usually in the cytosol fraction.

An aliquot of cytosol is incubated with an estrogen receptor ligand. Any ligand which binds to the receptor can be used. The only limitation is that the ligand-receptor complex must be detectable by some physical, chemical or immunological technique. For example, the procedures using radioactivity, fluorescence, immunological and UV and light wave detectors are all acceptable. In the preferred embodiment radiolabelled estradiol, either $^{3}$H-estradiol-17$\beta$ or $^{125}$I-iodoestradiol-17$\beta$ are used. In the preferred embodiment to determine the total number of estrogen binding receptors, the aliquot of cytosol is incubated with a saturating concentration, for example, about 10-20 nM, of radiolabelled estradiol.

The incubation of the cytosol with about 10-20 nM of radiolabelled estradiol is not time restricted. Satisfactory measurements have been made with incubation time from about 30 minutes to 24 hours. In the preferred embodiment the incubation time is usually 1-2 hours at about 0°C. However, to measure occupied estrogen receptors, estradiol exchange reactions for about 30 minutes at about 25-30°C may be employed. The temperature is maintained with a cooling unit, ice bath, or cold room. DNA and a urea buffer are added to the cytosol. The order or sequence of mixing the cytosol with urea buffer and DNA is not important. For example, the cytosol can be mixed with the DNA already suspended in a urea buffer or the cytosol can be incubated with radiolabelled estradiol in the presence of DNA and then combined with the urea buffer.

The concentration of urea required to promote maximal interaction of the steroid-receptor complex with DNA is not critical. From Table 1, one skilled in the art can readily recognize that urea concentrations from about 1 to 6 M are adequate.

### TABLE 1 – INTERACTION BETWEEN ESTROGEN RECEPTOR (ER) AND DNA AS A FUNCTION OF UREA CONCENTRATION

| E.G. | UREA CONCENTRATION | % ER BOUND TO DNA |
|---|---|---|
| | 1 M UREA | 48%-62% (n=5) |
| | 2 M UREA | 83%-92% (n=6) |
| | 4 M UREA | 90% (n=6) |
| | 6 M UREA | 100% (n=6) |

In a preferred embodiment about 2M urea is used. Urea concentrations from about 1 M to about 6 M have been used successfully at the highest concentrations of cytosol protein, about 20 mg/ml, usually ever encountered with clinical tumour specimens. When the concentration of cytosol is lower, the urea concentration can also be lowered. Urea concentration as low as about 1 M have been used. The urea solution used to promote the interaction of estrogen-receptor with the DNA is simply homogenization buffer containing about 1 M to about 6 M urea. The homogenization buffer is used for convenience. The EDTA, DTT and/or glycerol in the homogenization buffer are not specifically required for the urea-induced DNA-binding of estrogen receptor. A buffer using about 10-50 mM phosphate buffer (pH 7.4) can also be used. One skilled in the art will recognize that the buffer is not a necessary component and any equivalent solution will work.

The quantity of DNA is not critical as long as saturating concentrations of DNA are present. Additionally, the type of DNA is not critical. Both single stranded and double stranded DNA work equally well. The DNA can be free in solution or immobilized. The specific mode of DNA immobilization is not critical. Single-stranded DNA-agarose, double-stranded DNA-cellulose and DNA absorbed directly to filters or nitrocellulose tubes can be used. For convenience in measurement, immobilized DNA has been used in the preferred embodiment. Furthermore, there is no evidence for sequence specificity and any DNA may be used. Commercially available calf thymus DNA has been used. It can be seen in Table 2 that the concentration of DNA required is essentially linear from about 50 microliters cytosol to about 2 ml cystosol.

TABLE 2 - CONCENTRATION OF DNA REQUIRED FOR INTERACTION WITH ESTROGEN RECEPTOR (ER) AS A FUNCTION OF CYTOSOL VOLUME

| MICRO LITRES OF CYTOSOL ADDED TO 0.4 ML DNA-AGAROSE (0.27 mg DNA) | % ER BOUND TO DNA |
| --- | --- |
| 50 | 3329 cpm |
| 100 | 6628 |
| 200 | 12407 |
| 400 | 26621 |
| 600 | 42754 |
| 800 | 55319 |
| 1000 | 66195 |
| 2000 | 121227 |

Thus, at the levels used in the preferred embodiment, very low amounts of tissues or levels of estrogen receptor can be detected.

In a preferred embodiment, about 0.2-1.0 ml aliquot of DNA-agarose, which is about 0.6-0.7 mg DNA/ml swollen gel, is normally satisfactory for up to about a 2 ml aliquot of cytosol with about a 20 mg protein ml concentration. This amount of cytosol is about equivalent to 500 Fmoles of estrogen receptor/mg cytosol protein.

The urea-promoted DNA-binding assay is direct for the estrogen receptor and is not time dependent. The interaction probably occurs instantaneously upon combining the ligand-receptor complex, urea and DNA. The procedure itself limits examining the actual rate. The earliest measurement to get accurate results is at about 15 minutes. Table 3 shows the % estrogen receptor which binds to DNA.

## TABLE 3 — INTERACTION BETWEEN ESTROGEN RECEPTOR (ER) AND DNA AS A FUNCTION OF TIME IN UREA

| LENGTH OF EXPOSURE OF ER TO UREA PRIOR TO ASSESSMENT OF DNA BINDING | %ER BOUND TO DNA |
|---|---|
| < 15 minutes | 86-89% |
| 30-45 minutes | 100% |
| 90-120 minutes | 100% |

One skilled in the art will recognize that the binding is rapid and complete in a very short time. After a 15 minute to 45 minute incubation of the cytosol with DNA in the urea buffer, non-specific estrogen binding proteins and free steroid are removed by decantation and washing. The washing procedure involves the continued use of the urea buffer to either rinse or re-suspend the immobilized DNA. A volume of washing buffer equal to 2-4 ml is normally sufficient to reduce the background of free steroid (e.g. $^3$H-estradiol-17$\beta$) and non-specific binding proteins to below 1% of the estrogen-receptor present. when the DNA is immobilized directly to the inside of the test tube (12 $\times$ 75 mm nitrocellulose), the washing procedure simply involves rinsing the inside of the tube. When the DNA is immobilized to agarose or cellulose (or similar stationary phase gel or matrix) the washing procedure requires re-suspension of the DNA into urea washing buffer, repelleting by low speed (up to 10,000 $\times$ g) centrifugation (1-5 min) and decanting. This procedure should be repeated 2-3 times using 2-3 ml of wash buffer each time or until background radioactivity is less than 1% of the receptor activity present.

Absence of significant interaction between free steroid and DNA, was shown by adding labelled total free steroid (84286 CPM). Only a very small amount of this labelled material was not eluted (256 CPM). This is less than 0.4% background. Similar data are shown in Table 4 for non-specific estrogen binding protein.

TABLE 4

ABSENCE OF SIGNIFICANT INTERACTION BETWEEN NON-RECEPTOR ESTROGEN-BINDING PROTEINS (NSB) AND DNA NSB ACTIVITY ADDED:     83695 (INCLUDES FREE STEROID)
NSB ACTIVITY ELUTED:     243
PERCENTAGE:     0.3%
RANGE:     0.15 - 0.7% (n = 5)

Again, one skilled in the art will readily recognize that minimal to no detectable amounts of radioactivity were recovered in DNA-bound fraction. These results demonstrate that it is only the estrogen-estrogen receptor complex which is binding to DNA. One skilled in the art will readily recognize many advantages of this high selectivity including the use of very small tissue samples and accurate measurements of low quantities of estrogen receptors.

Further experiments examined the ligand-estrogen receptor interaction with DNA under various previously reported studies. In Table 3 it is clearly evident that the present urea-promoted DNA-binding assay is very much superior to any other method. Furthermore, it is quite evident that the other procedures by themselves cannot be utilized for assaying estrogen receptors.

Alternatively the cytosol could be incubated with $^3$H-estradiol and non immobilized (free) DNA simultaneously. Upon subsequent addition of urea to promote DNA binding, the mixture can be filtered through one of several commercially available DNA-binding filters. These will bind DNA along with its bound proteins (in this case the estrogen-receptor protein complex).

The radiolabelled estrogen-receptors which are bound to the immobilized DNA at this point can be detected directly by a gamma counter if $^{125}$I-iodoestradiol-17$\beta$ was used as the radioligand. If $^3$H-estradiol was used to label the receptor, the DNA bound $^3$H-estradiol-receptor complexes must be eluted. For example only, and not by limitation, the labelled receptor complex can be quantitatively eluted from the DNA using homogenizing buffer containing 6 M urea and 0.4 M KCl. Alternatively, ethanol can be used to extract the $^3$H-estradiol-17$\beta$. One skilled in the art will readily recognize that other methods and solutions can be used to remove the radiolabel for detection. $\beta$-emitting radioactivity is then quantitatively assessed using a liquid scintillation spectrophotometer.

The original cytosol protein concentration is determined by the standard methods of protein assay.

The results are expressed as femtomoles of estrogen receptor calculated directly from the DNA-bound radioactivity per mg of cytosol protein.

The quantity of functional estrogen receptor proteins in tumour tissues reflects the potential ability of that tumour to respond (e.g. regress) to either additive (e.g. antiestrogens) and/or ablative (e.g. ovariectomy, adrenalectomy) hormonal therapies. In the absence of clinical biochemical evidence for estrogen receptor, patients are often managed or treated by non-hormonal therapies. The presence and quantity of estrogen receptor often allows the prediction for a more extended disease-free interval (1,2). As shown in Table 5 the UDB assay is clearly as good as existing assays for receptor measurement.

TABLE 5 - APPLICATION OF UREA-INDUCED DNA-BINDING ASSAY (UDB) TO THE DIRECT QUANTIFICATION OF ESTROGEN RECEPTOR IN HUMAN TUMOUR TISSUES.

RECEPTOR QUANTITY

| ASSAY | UTERINE ADENOCARCINOMA | BREASt CARCINOMA |
|---|---|---|
| HAP | 8181 cpm | 4509 cpm |
| UDB | 8253 cpm (100%) | 4387 cpm (97%) |

The urea-induced DNA-Binding assay affords a rapid and direct, solid phase approach to the quantification of estrogen receptor proteins in both normal and neoplastic tissues. One skilled in the art will readily recognize that this procedure can be automated by a variety of methods using one or more of the above procedures.

Claims

1. A direct assay for the detection of estrogen receptors, comprising the steps of:

(a) adding estrogen receptor ligand, estrogen receptor, DNA and urea in amounts sufficient to form a mixture comprising a ligand-receptor-DNA complex; and

(b) determining the amount of said ligand-receptor-DNA complex as an indication of the amount of estrogen receptors.

2. The assay of Claim 1, wherein cytosol from endocrine target tissue is combined with an estrogen receptor ligand in amounts sufficient to form said ligand-receptor complex.

9

3. The assay of Claim 2, wherein the endocrine target tissue is selected from the group consisting of normal breast tissue, normal uterus tissue, breast tumour tissue and uterine tumour tissue;

4. The assay of Claim 2 or 3, wherein the estrogen receptor ligand is selected from the group consisting of estrogens and anti-estrogens.

5. The assay of Claim 2, 3 or 4, wherein the estrogen receptor ligand is radiolabelled estradiol.

6. The assay of any preceding Claim, wherein the urea is at least about 1 M.

7. A direct assay for the detection of estrogen receptors, comprising the steps of:

(a) incubating cytosol from endocrine target tissue with radiolabelled estradiol in amounts sufficient to form an estradiol-estrogen receptor complex;

(b) adding urea and DNA in amounts sufficient to form an estradiol-estrogen receptor-DNA complex; and

(c) determining the amount of said estradiol-estrogen receptor-DNA complex as an indication of the amount of estrogen receptors.

8. The assay of Claim 7, wherein:

(a) the cytosol comprises at least about 0.5 mg of protein/ml;

(b) the amount of radiolabelled estradiol is at least about 0.5 nM;

(c) the incubation is at about 0 °C for at least about 30 minutes; and

(d) the urea is in a concentration of at least about 0.5 M.

9. The assay of any preceding claim, wherein the DNA is selected from the group consisting of free single stranded DNA, immobilized single stranded DNA, free double stranded DNA and immobilized double stranded DNA.

10. The assay of Claim 9, wherein said DNA is selected from the group consisting of free single stranded DNA and free double stranded DNA and comprising the further step of passing said mixture through a DNA binding filter to separate said estradiol-estrogen receptor-free DNA complex.

11. An assay according to any preceding claim, which is automated.